# EUROPÄISCHE PATENTSCHRIFT

(11) **EP 2 287 091 B1**
(45) Veröffentlichungstag und Bekanntmachung des Hinweises auf die Patenterteilung: **04.06.2014**
(21) Anmeldenummer: 10008074.6
(22) Anmeldetag: 03.08.2010
(51) Int. Cl.: B65F 1/14, A61B 19/02

(54) **Vorrichtung und Aufnahme von zu entsorgenden Gegenständen für einen Operationssaal**
Device and holder for objects to be removed from an operating theatre
Dispositif et réception d'objets devant être recyclés pour une salle d'opération

(30) Priorität: 14.08.2009 DE 102009037315
(43) Veröffentlichungstag der Anmeldung: 23.02.2011
(73) Patentinhaber: Karl Storz GmbH & Co. KG, 78532 Tuttlingen (DE)
(72) Erfinder: Hiltl, Christoph, 78532 Tuttlingen (DE)
(74) Vertreter: Fugmann, Winfried

(56) Entgegenhaltungen:
- WO-A1-2009/030877
- WO-A2-2005/029286
- DE-A1- 19 612 602
- US-A1- 2002 196 150

## Beschreibung

Die vorliegende Erfindung betrifft eine Vorrichtung zur Aufnahme von zu entsorgenden Gegenständen.

Zu entsorgende Gegenstände umfassen insbesondere chirurgische Instrumente, medizinisches Verbrauchsmaterial und Sterilgutverpackungen, also Behältnisse, in denen ein Sterilgut bis zum unmittelbaren Verbrauch bleibt.

Eine Vorrichtung zur Aufnahme von zu entsorgenden Gegenständen im Bereich der Stationen eines Krankenhauses, ist aus der US 2002/0196150 A1 bekannt. Diese wird auf den Stationszimmern eines Krankenhauses eingesetzt um die Entsorgung mit dem Krankenhauspersonal zu korrelieren. Dabei wird nur kontrolliert, ob Müll in die Entsorgungseinheit verbracht worden ist, in dem ein Emitter und ein Detektor den Durchgang eines Gegenstandes durch eine Öffnung prüfen. In einer zusätzlichen Ausgestaltung ist vorgesehen, dass ein Anhänger an den Verpackungsmüll angebracht ist, der dann ähnlich wie die Plakette des Krankenhauspersonals durch einen Sensor erkannt wird, wenner in der Nähe der Müllentsorgungseinheit ist.

Aus der WO 2005/029286 ist bekannt, dass eine Müllsortiereinrichtung über einen Scanner verfügt. An diesem wird der zu entsorgende Müll gescannt. Anschließend wird ein Müllbehälter zugewiesen, dazu wird der entsprechende Behälter zugänglich gemacht.

Eine Vorrichtung zur Aufnahme von zu entsorgenden Gegenständen für einen Operationsaal ist aus der DE-OS 2146823 bekannt.

Chirurgische Instrumente und Sterilgüter wie beispielsweise Tupfer und Operationstücher, die bei Operationen benötigt werden, werden nach Gebrauch direkt in einen solchen Abfalleimer geworfen oder in anderen Bereitstellungsbehältern, beispielsweise Siebkörben, abgelegt. Für die medizinische Dokumentation ist es dann üblich, die verwendeten Artikel in einer OP-Dokumentationsliste zu dokumentieren. Diese Arbeit wird vollständig manuell durchgeführt. Sie ist sehr arbeits- und zeitaufwändig, und es bedarf hierzu eines hoch spezialisierten Pflegepersonals, das die einzelnen Instrumente, Verbrauchsgüter und Sterilgüter erkennt, und beim manuellen Dokumentieren keine Übertragungsfehler macht.

Die Komplexität der Sterilgutverwaltung erfordert mittlerweile ein Sterilgutmanagement, das in der Lage ist, alle Abläufe im Zusammenhang mit Sterilgut in einem Krankenhaus schnell zu erfassen und zu beherrschen. Das Prinzip der Identifikation und Rückverfolgbarkeit ist dabei nur eine der wesentlichen Aufgaben.

Der vorliegenden Erfindung liegt die Aufgabe zugrunde, eine Vorrichtung zur Aufnahme zu entsorgenden Gegenständen so weiterzubilden, dass eine verbesserte Dokumentation möglich ist.

Diese Aufgabe wird durch eine Vorrichtung zur Aufnahme von zu entsorgenden Gegenständen mit den Merkmalen des Patentanspruchs 1 gelöst. Die abhängigen Ansprüche zeigen Weiterbildungen der Erfindung.

Die erfindungsgemäße Vorrichtung umfasst einen mit einer Öffnung versehenen Behälter und eine Identifizierungseinheit, die im Öffnungsbereicht des Behälters angeordnet ist.

Auf Grundlage der erfindungsgemäßen Vorrichtung ist es möglich, einen mit einem und/oder mehreren Datenträger versehenen Gegenstand sicher zu erfassen und Daten des Datenträgers auszulesen, wenn dieser dem Detektionsbereich der Identifikationseinheit zugeführt wird. Die hierdurch gegebenen, wesentlichen Vorteile sind: eine Zeitersparnis bei der Erfassung der Gegenstände, weniger Fehler in der Dokumentation und die Vorrichtung ist leicht und intuitiv zu benutzen und in Folge dessen bedarf es keines speziell dafür geschulten Personals.

Die erfindungsgemäße Identifizierungseinheit weist wenigstens einen Lesekopf auf, der einen durchgehenden Detektionsbereich zeigt, welcher die Behälteröffnung vollständig abdeckt und erfasst und sich insbesondere horizontal, vorzugsweise parallel zur Oberfläche der Behälteröffnung erstreckt und dem Profil des Öffnungsumfangs des Behälters entspricht. Der Detektionsbereich entspricht dabei insbesondere der Behälteröffnung Auf diese Weise wird sichergestellt, dass jeder mit einem Datenträger versehener Gegenstand, welcher in den Behälter eingebracht wird, verlässlich erfasst wird und die Daten des Datenträgers entsprechend ausgelesen werden können. Darüber hinaus wird durch die gewählte Ausbildung der Identifizierungseinheit auch die Möglichkeit der Fehldetektion ausgeschlossen, nämlich dass unerwünschterweise auch außerhalb des Behälters befindliche Gegenstände, die in unmittelbarer Nähe der Identifizierungseinheit entlang geführt werden, erfasst werden. Dabei wird durch die Identifizierungseinheit die Erfassung ausschließlich der tatsächlich zu entsorgenden Gegenstände gewährleistet und in einer bevorzugten Ausführung wird zusätzlich deren Identifizierung vorgenommen. Neben dem erfassten Datum können zusätzlich und alternativ dann weitere Daten zu den identifizierten Gegenständen dokumentiert und einer weiteren Auswertung zugeführt werden.

In der erfindungsgemäßen Vorrichtung weist die Identifizierungseinheit zwei oder mehrere Leseköpfe auf. Diese sind bevorzugt so angeordnet und ausgerichtet, dass sich die jeweiligen Detektionsbereiche der aneinander angrenzenden und/oder gegenüberliegend angeordneten Leseköpfe überlappen und gemeinsam einen durchgehenden Detektionsbereich bilden. Eine solche Konfiguration hat den Vorteil, dass eine zuverlässige Erfassung von mit Datenträgern versehenen Gegenständen gewährleistet ist. Gerade bei Gegenständen, deren Datenträger sich für einen einzelnen Lesekopf in einer nicht lesbaren Stellung befindet und/oder störende abschirmende Komponenten aufweisen, wird erfindungsgemäß trotzdem eine sichere Erfassung erreicht.

Ein oder mehrere der Leseköpfe der erfindungsgemäßen Vorrichtung können bevorzugt als Barcodelesegerät und/oder RFID-Lesegerät ausgebildet sein. Diese Mittel sind dazu geeignet, die Informationen aus Datenträgern, wie zum Beispiel Barcodeetiketten oder RFID-Datenträger - auch RFID-Tags genannt -, berührungsfrei auslesen zu können. Dies erfolgt weitgehend unabhängig von der Verschmutzung der zu entsorgenden Gegenstände. Weitere Vorteile von RFID-Datenträger und RFID-Lesegeräten liegen in der höheren Speicherkapazität, der schnelleren Geschwindigkeit beim Auslesen und Identifizieren gegenüber den üblichen anderen Datenträgern und zugehörigen Lesegeräten wie zum Beispiel Barcode-Systemen. Auch können die Daten direkt und exakt erfasst werden. Die Daten können insbesondere aus der Datengruppe Produktname, Artikelbezeichnung, Lotnummer, Verwendbarkeits-/Verfallsdatum, Seriennummer entnommen sein.

Darüber hinaus stellen solche Lesegeräte standardisierte Bauteile mit einer hohen Erfassungsgenauigkeit dar, mit denen sich eine kostengünstige Vorrichtung realisieren lässt. Der Vorteil eines Barcodes bzw. Barcodelesesystems liegt insbesondere darin, dass diese Technologie im Bereich der automatisierten Kennungssysteme ausgereift und bereits seit vielen Jahren in vielen Branchen weit verbreitet ist. Auch können die zu entsorgenden Gegenstände auch auf einfache und verlässliche Weise beschriftet sein und werden und damit besonders einfach erfasst und einer Dokumentation zugeführt werden.

Mittels der Leseköpfe, die bevorzugt als Barcodelesegerät und/oder RFID-Lesegerät ausgebildet sind, können Daten zum zu entsorgenden Gegenstand aus dessen Datenträger ausgelesen und später dokumentiert werden. Im Rahmen einer weiteren Auswertung können diese Daten dann mit weiteren Daten insbesondere aus einer Datenbank ergänzt werden und entsprechend einer weiteren Auswertung und Dokumentation zugeführt werden. Dies kann zum Beispiel zu einer Materialflussinformation führen. Im Rahmen einer fakultativen Identifikation mittels der Identifizierungseinheit kann anhand der erfassten Daten auch eine Identifikation durch Vergleich mit vorabgespeicherten Gegenstandsdaten erfolgen, wobei diese bevorzugt in einer abgesetzten Datenbank oder in eine der Identifizierungseinheit angeordneten Datenbank abgelegt sind. In diesem letzten Fall wird die Datenbank regelmäßig oder situationsbedingt anhand einer abgesetzten Datenbank insbesondere im zentralen Rechner des Krankenhauses aktualisiert. Hierdurch lässt sich eine sehr aktuelle und verlässliche Dokumentation erreichen.

Eine weitere Ausgestaltung der erfindungsgemäßen Vorrichtung sieht vor, dass die Identifizierungseinheit über eine Kommunikationsverbindung mit einer von ihr abgesetzten Auswerteeinheit verbunden ist und die Übertragung der durch die Identifizierungseinheit ausgelesenen Daten an die Auswerteeinheit vorzugsweise unter Verwendung dieser Kommunikationsverbindung erfolgt. Zur Bewerkstelligung eines lokalen Datentransfers kann die Identifizierungseinheit dabei physisch über ein Datenkabel mit der Auswerteeinheit verbunden sein. Die Verwendung eines Datenkabels birgt jedoch die Gefahr des Stolperns über das Kabel oder das unbeabsichtigte Trennen des Kabels von der Identifizierungseinheit und/oder Auswerteeinheit durch Stoß und/oder Zug.

Alternativ und bevorzugt erfolgt der Datentransfer drahtlos, beispielsweise über eine IR-oder Bluetooth-Verbindung, die sich insbesondere bei häufiger Ortsveränderung der erfindungsgemäßen Vorrichtung als nützlich erweist.

Die Übertragung der Daten kann in Echtzeit d.h. während einer laufenden Identifizierung und des Auslesens eines an einem Lesekopf herangeführten mit einem Datenträger versehenen Gegenstandes ohne wesentliche Verzögerung erfolgen. Hierdurch wird es auf vorteilhafter Weise möglich, auf besondere Datenspeichersysteme zur Zwischenspeicherung innerhalb der Identifizierungseinheit zu verzichten und diese entsprechend klein und preiswert herzustellen.

Alternativ hierzu ist es auch möglich, im Bereich der Identifizierungseinheit oder der Kommunikationsverbindung Datenspeichereinrichtungen vorzusehen, durch welche die Daten zunächst gespeichert, und anschließend oder zumindest mit einem Zeitversatz ausgelesen und weiter geleitet werden können.

Es ist auch möglich, eine einfache Vorverarbeitung der erfassten Daten noch im Bereich der Identifizierungseinheit durchzuführen. So können im Rahmen dieser Vorverarbeitung diese Daten für eine einfache Nachverarbeitung normiert werden.

Bei einer vorteilhaften Ausführungsform der erfindungsgemäßen Vorrichtung kann die Auswerteeinheit dabei beispielsweise ein PC-Arbeitsplatz mit einem Monitor und einer Tastatur oder ein mobiles Handgerät sein. So kann mittels der Tastatur beispielsweise Patientenidentifizierungsinformationen zusätzlich eingegeben werden, die eine eindeutige Zuordnung der erfassten Gegenstände zum Patienten gewährleistet.

In einer besonders zweckmäßigen und bevorzugten Ausgestaltung der erfindungsgemäßen Vorrichtung ist die Auswerteeinheit mit einem Touch-Screen zu einer Einheit verbunden, die die Bearbeitung der eingelesenen in Verbindung mit einer Eingabe durch Berührung der Anzeigefläche und/oder durch entsprechende Eingabe von Bedienungs- und Steuerbefehlen ermöglicht.

Die Auswerteeinheit kann dabei innerhalb des Operationssaales vorzugsweise in unmittelbarer Nähe des Operationstisches und/oder in einem Bereich des Operationssaales positioniert werden. So können die zu entsorgenden Gegenstände unmittelbar nach oder schon während des Eingriffs auf kurzem Weg der Erfassung und erweiterten Dokumentation zugeführt werden können.

In einer besonders vorteilhaften Ausführungsform ist die Auswerteeinheit außerhalb des Operationssaales angeordnet. Dies hat den Vorteil, dass die Erfassung und Dokumentation in einem nicht sterilen Raum durchgeführt werden kann und in Folge dessen aufgrund der nicht gegebenen, potentiellen Kontaminationsgefährdung des Patienten durch das Bedienpersonal, das aus hygienischer Sicht keine, für einen Operationssaal zwingend vorgeschriebene, Schutzkleidung tragen muss. Des Weiteren muss die Auswerteeinheit nicht aus speziellen, aufwändigeren und damit teuren sterilisationsfesten Materialien hergestellt sein.

Es ist vorgesehen, dass die Auswerteeinheit zunächst die Roh-Daten und/oder Roh-Datensätze von einer Identifizierungseinheit empfängt und dass diese dann beispielsweise in ein Standardtabellenkalkulationsprogramm eingelesen und abspeichert werden, wobei die Datensätze Informationen aus der Gruppe Produktname, Artikelbezeichnung, Lotnummer, Verwendbarkeits-/Verfallsdatum, Seriennummer enthalten, die selektiv in die Tabellenfelder geordnet werden. Nach erfolgter Datenzuordnung können dann Auswertungsergebnisse automatisch generiert werden. Beispielsweise kann eine Inventurdatei bzw. eine Materialflußinformation erzeugt werden.

Eine in der Auswerteeinheit gespeicherte Inventurdatei beinhaltet somit z.B. die Anzahl der Gegenstände, die für den Patienten während des Eingriffs bereitgestellt und verwendet bzw. eingesetzt wurden. Die erzeugte Inventurdatei kann dann mit einer Vorherigen, die zu Beginn des Eingriffs erstellt wurde, verglichen werden. Je nach angewandten Abfragen kann beispielsweise mittels einer OP-Dokumentationsliste Auskunft darüber geben werden, ob und/oder wie viele Gegenstände während des Eingriff tatsächlich eingesetzt wurden. Insbesondere kann damit sichergestellt werden, ob alle eingesetzten Instrumente und sonstigen Materialien am Ende der Operation vollständig und aktuell erfasst sind. Diese Information ist im Interesse der Patienten wichtig.

In einer bevorzugten Ausführung ist die Auswerteeinheit mit der zentralen Datenbank vorzugsweise des Krankenhauses auch Krankenhausinformationssystem, KIS, genannt, elektronisch beispielsweise via GPRS/WLAN verbunden. Die in der Auswerteeinheit generierten Auswertungsergebnisse können dann beispielsweise insbesondere regelmäßig oder situationsbedingt gesteuert, insbesondere nach einem abgeschlossenen Eingriff, an die zentrale Datenbank des Krankenhauses geschickt und/oder von dieser abgerufen werden und im Rechner der zentralen Datenbank abgespeichert und/oder weiterverarbeitet werden.

Durch die jederzeit mögliche Datenerfassung können Materialflussinformationen innerhalb des Krankenhauses zu jeder Zeit abgefragt, mitgeteilt bzw. angezeigt werden.

Aufgrund der Tatsache, dass regelmäßig im Rechner der zentralen Datenbank die kennzeichnenden Daten eines jeden Gutes zum Zeitpunkt des Eingangs in das Sterilgutlager des Krankenhauses und auch zum Zeitpunkt der Erfassung im Operationssaal registriert werden, ist es insbesondere möglich, unter Verwendung eines elektronischen Bestands-Mengen- und Wertfeststellungsverfahrens eine permanente Bilanzierung des Lagerbestandes durchzuführen.

So kann beispielsweise der jeweils aktuelle Lagerbestand und die Bewegung im Lager jederzeit mittels einer Grafik oder Inventurliste dargestellt werden. Zum anderen besteht auch die Möglichkeit, die verbrauchten und/oder eingesetzten Gegenstände automatisch abzuleiten und bestimmten Verbrauchsstellen wie beispielsweise einzelnen Operationssäle zuzuordnen, um schließlich beispielhaft eine entsprechende patientenbezogene Kostenerfassung durchzuführen.

Die erfindungsgemäße Vorrichtung weist regelmäßig einen Behälter mit einer nach oben weisenden Öffnung, einer Standfläche und einen Innenraum auf, in den die zugeführten Gegenstände unter Mitwirkung der Schwerkraft aufgenommen werden. Der Behälter kann dabei aus unterschiedlichen Materialien gefertigt sein. Insbesondere ist es zweckmäßig die Materialien derart auszuwählen, dass sie autoklavierbar sind, also einer Wasserdampfbehandlung unter Druck von bis zu etwa 134°C ohne Beschädigung widerstehen können. Eine Autoklavierbarkeit ist insbesondere dann unumgänglich und daher für die erfindungsgemäße Vorrichtung von großem Vorteil, wenn der Behälter beispielsweise mit Blut und/oder Keimen kontaminiert sein sollte. Eine Autoklavierung erlaubt dann eine Wiederbenutzung des Behälters und somit eine höhere Lebensdauer des Behältnisses insgesamt, während nicht autoklavierbare Behälter nach jeder Kontamination mit Keimen als medizinischer Sondermüll kostspielig entsorgt werden müssen.

In einer weiteren vorteilhaften Ausgestaltung ist der Behälter gegenüber dem Aufbewahrungs- und/oder Arbeitsort auf beispielsweise Rollen verschiebbar oder mittels beispielsweise eines Wiegegestells verschwenk- bzw. kippbarbar. Beispielsweise um 10-15°. So kann der Behälter beispielsweise auf Rollen gelagert zunächst ganz und gar unproblematisch hin zum Einsatzort bewegt werden und dort vom Benutzter bei Bedarf oder Notwendigkeit selbst völlig problemlos durch Heranschieben und Verschwenken in die jeweilige Betriebs- bzw. Überführungsposition verbracht werden, so dass ein bequemes und ergonomisches Arbeiten möglich wird. Hierdurch wird sichergestellt, dass die erfindungsgemäße Vorrichtung immer an dem gewünschten Ort zur Verfügung steht und dadurch eine effiziente Dokumentation der Gegenstände erreicht werden kann.

Eine weitere, vorteilhafte Ausführungsform der erfinderischen Vorrichtung besteht darin, dass der Innenraum des Behälters Trennwände aufweist und je nach Anzahl der Trennwände der Innenraum in mindestens zwei Kompartimente unterteilt ist. Auf diese Weise ermöglicht dieser Behälter, dass wenigstens zwei Kategorien von Gegenständen sortiert und entsprechend ihrer Sortierkriterien einem der Kompartimente zugeführt werden können. So können etwa alle Gegenstände, die wie Katheter und Schläuche aus PVC bestehen, sortiert, gesammelt und schließlich dem Recycling zugeführt werden. Die entsorgten und in verschiedene Kompartimente sortierten Gegenstände werden dabei bevorzugt selektiv dokumentiert, so dass eine differenzierte Auswertung z.B. nach dem Umfang der Gegenstände je Kategorie ermöglicht ist.

In einer weiteren, bevorzugten Ausführungsform kann ein einzelnes Kompartiment so ausgebildet werden, dass aufgrund der vorgegebenen, während der Operation eingesetzten Gegenstände, eine Anpassung an unterschiedliche Anwendungserfordernisse möglich ist. So kann ein Kompartiment für die selektive Aufnahme von Spritzen mit einer besonders durchstichsicheren Wandung ausgebildet sein, um möglich Verletzungen bei der Herausnahme des Kompartiments zu verhindern. Des Weiteren können sich die Aufnahmevolumina von Kompartimente bzw. die Durchlassquerschnitte ihrer Öffnungen unterscheiden. Dies kann entweder durch unterschiedliche Zusammensetzung modulartiger Bauteile bei der Fertigung und/oder nachträglichen Verstellbarkeit erreicht werden. So können dann Gegenstände, wie beispielsweise wieder verwendbare chirurgische Instrumente, mittels der Sortierung zusammengestellt werden, um schließlich im Wege der Instrumentenwiederaufbereitung einem Sterilisierungsverfahren zugeführt werden zu können.

In einer weiteren praktischen Ausführungsform sind der Behälter und/oder ein Kompartiment im Inneren mit einem wieder herausnehmbaren Plastiksack ausgekleidet. Vorzugsweise ist dieser mit individualisierten Kennzeichen versehen, die die Dokumentation ermöglichen und/oder die Logistik und/oder Entsorgung verbessern.

Darüber hinaus kann ein Kompartiment auch als Plastiksack/Kunststoffbeutel ausgeführt sein und für Gegenstände Anwendung finden, die nicht recyclebar sind und der Abfallbeseitigung zugeführt werden.

Eine weitere Ausbildung der erfindungsgemäßen Vorrichtung betrifft einen Behälter mit einer nach oben weisenden Öffnung, welche mit einem Deckel versehen ist, der die Öffnung abdeckt. In jedem Fall kann die Öffnung des Behälters mit einem Deckel versehen sein, welche die Öffnung abdeckt. Es kann allerdings auch vorgesehen werden, dass mehrere Kompartimente jeweils mit einem Deckel versehen sind wobei nicht jeder mit einem Deckel versehen muss sein. Hierdurch ist definiertes Einbringen in den Behälter bzw. die Kompartimente gewährleistet, was Fehleinbringungen verhindert und damit die Datenqualität der Dokumentation verbessert.

Das Öffnen und Schließen eines und/oder mehrere Deckel kann manuell erfolgen. Alternativ und bevorzugt erfolgt das Öffnen und Schließens durch elektronische Schaltungen auf Basis eines von der Identifizierungseinheit her empfangenen Signals für die Detektion von zu entsorgenden Gegenständen. Die Steuerung ist so konfiguriert, dass der Behälter und/oder das Kompartiment nach Aufnahme des Gegenstandes verschlossen wird und somit ein unbeabsichtigtes Nachwerfen bzw. Zuführen eines sortenfremden Gegenstandes in den Behälter und/oder in das Kompartiment verhindert wird.

Für die erfindungsgemäße Vorrichtung ist vorzugsweise auch eine im Öffnungsbereich des Behälters angeordnete, bedarfsgesteuerte Sortiervorrichtung vorgesehen, welche gerade die Informationen der Identifizierungseinheit zu ihrer Steuerung nutzen kann. Dabei werden von der Identifizierungseinheit Daten aus dem Datenträger des Gegenstandes ausgelesen, die insbesondere Angaben über die Sortenzugehörigkeit eines Gegenstandes zulassen. Dabei wird eine Sortenzugehörigkeit anhand folgender Kriterien z.B. Größe, Form, Werkstoff, Art, usw. bestimmt.

Entsprechend der Sortieraufgabe wird dann der Gegenstand beispielsweise mechanisch ausgetragen und einem entsprechenden Kompartiment zugeführt. Somit überträgt der Gegenstand selbst die für die optimale Entsorgung erforderlichen Daten an die Sortieranlage. Es können vorteilhaft Gegenstände dabei automatisch dokumentiert, sortiert und eindeutig zusammengeführt werden, die sich in der Form und/oder in der Art ihrer Wiederverwertung gleichen, wie beispielsweise geöffnete Produktverpackungen oder chirurgische Instrumente.

Das mechanische Austragen bzw. das Aussortieren der Gegenstände kann dabei beispielsweise von einer Weiche bewirkt werden, die um eine Drehachse gelagert ist und durch ihre eingenommene Stellung bestimmt, in welches Kompartiment die sortierten Produkte gelangen. Ebenso ist eine schwenkbare, schräg angeordnete Rutsche denkbar, über die die sortierten Gegenstände aufgrund der Schwerkraft selektiv ausgeleitet werden und schließlich in ein Kompartiment fallen. Des Weiteren kann ein Ausschieber oder eine Blasdüse für das Ausleiten auf einer Rutsche vorgesehen sein. Dies ermöglicht eine sehr verlässliche Selektion der Gegenstände insbesondere im Hinblick auf die Logistik und Entsorgung und daraus folgend eine aussagekräftige Dokumentation.

Bei einer weiteren bevorzugten Ausführungsform der erfindungsgemäßen Vorrichtung weist die Vorrichtung ein Handgerät auf, das über eine Verbindungseinrichtung insbesondere funkbasiert mit der Identifizierungseinheit verbunden ist. Hierdurch wird das Auslesen der erfassten, ausgewerteten oder gespeicherten Daten insbesondere aus der Identifizierungseinheit ermöglicht. Zusätzlich können für die Identifikation notwendige oder hilfreiche gegenstandsbezogene Daten über das Handgerät eingegeben und z.B. an die Identifizierungseinheit weitergegeben werden, und diese Daten dann bei der weiteren Identifikation bzw. Auswertung verwendet werden.

Auch kann ein Handlesegerätes zum Erfassen und Auslesen von Datenträgern über die Verbindungseinrichtung anschließbar sein. Vorzugsweise wird die Datenverbindung zwischen Handgerät, Handlesegerätes bzw. Verbindungseinrichtung drahtlos ausgebildet. Diese Ausführungsformen sind geprägt durch die Mobilität der Handgeräte bzw. Handlesegeräte, die durch die für den Benutzer verbesserte Handhabung eine verbessere Datenqualität ermöglicht.

Gemäß einer besonders vorteilhaften Ausführungsform der erfindungsgemäßen Vorrichtung ist diese mit einer Füllzustandsdetektionseinrichtung versehen, um anhand der erfassten Füllzustandsdaten und deren Dokumentation, den weiteren Logistikprozess der Entsorgung mit Zwischenlagerung, Transport und endgültiger insbesondere thermischen Entsorgung zu verbessern.

Mit der Füllzustandsdetektionseinrichtung werden das Gewicht und/oder das Volumen eingebrachter Gegenstände bestimmt. Zusätzlich kann auch das Gewicht und das Volumen eines Behälters und/oder Kompartiments mittels einer Füllzustandsdetektionseinrichtung beim Einbringen in den Behälter und/oder in ein Kompartiment gemessen werden. Auch kann gegebenenfalls daraus das Gesamtgewicht und/oder Gesamtvolumen aller eingebrachten Gegenstände berechnet werden. Der gemessene Gewichts- und/oder Volumenwert kann dann zur Anzeige gebracht werden. So kann der Füllzustand erkannt und z.B. abgespeichert und/oder angezeigt werden, ob und wann das maximale Füllvolumen des Behälters und/oder Kompartiments erreicht ist und der Behälter und/oder ein Kompartiment in Folge dessen geleert oder ausgetauscht werden muss.

Das Wiegen des eingeworfenen Abfalls kann beispielsweise durch eine unterhalb des Behälters und/oder Kompartiments angeordneten Wiegeplatte erfolgen, die zur Detektion der Gewichtsveränderung mit einem Gewichtsaufnehmer/-sensor versehen ist. Diese Ausführung ist besonders dann vorteilhaft einzusetzen, wenn der Abfall nach Einwurf in den Innenraum des Behälters und/oder Kompartiments über eine Fallhöhe fällt. Bei einer weiteren, vorteilhaften Ausführungsform ist die Wiegeplatte derart ausgeführt ist, dass sie mehrere, entsprechend der Anzahl der Kompartimente, Kraftaufnehmern aufweist und dass diese jeweils unter einem Kompartiment angeordnet sind. Dies hat den Vorteil, dass die Gewichtsveränderung eines Kompartiments individuell gemessen und angezeigt werden kann, insbesondere dann, wenn diesem Kompartiment ein Gegenstand zugeführt wurde.

In einer weiteren bevorzugten Ausführungsform ist vorgesehen, dass der Behälter und/oder ein Kompartiment zur Detektion des Volumens über mindestens einen Abstandssensor verfügt. Es ist besonders vorteilhaft, wenn der Abstandssensor unmittelbar unterhalb der vorgegebenen Füllhöhe des Behälters und/oder Kompartiments angeordnet ist, so dass mit dessen Hilfe automatisch und kontinuierlich die Füllhöhe des Behälters und/oder eines Kompartiments messbar ist und auch eine Überbefüllung des Behälters und/oder Kompartiments zuverlässig verhindert wird. Auch kann z.B. durch das Wiegen eine Verifikation der identifizierten Gegenstände von mit abgespeicherten Gegenstandsprofilen erfolgen. Hierdurch lässt sich die Sicherheit deutlich erhöhen.

Auch ohne ein Wiegen oder ohne eine Bestimmung des Volumens lässt sich eine Information über das Gewicht oder das Volumen der identifizierten Gegenstände anhand von in der Datenbank abgespeicherten Gegenstandsprofilen bestimmen und daraus der Füllzustand des Behälters bestimmen. Damit lässt sich eine verlässliche Dokumentation schaffen, welche Gegenstände in dem insbesondere vollen Behälter vorliegen und welche gegebenenfalls gemeinsam in einen Sack entsorgt werden. Vorzugsweise sind die aus dem Behälter entnommenen Säcke mit individualisierten Kennzeichen versehen, die die zusätzliche Dokumentation ermöglichen.

Bei einer weiteren praktischen Ausbildung der Erfindung ist vorgesehen, dass die Identifizierungseinheit Bestandteil eines insbesondere ringförmigen Aufsatzes ist, der auf einen Behälter abnehmbar und aufsetzbar angeordnet werden kann. Dieser ist vorzugsweise einstückig und selbsttragend ausgebildet. Dabei geht die Ringöffnung des Aufsatzes in die Öffnung des Behälters über und ermöglicht somit eine gute Identifikation mittels des Aufsatzes. Mit dieser erfindungsgemäßen Bauweise kann der Behälter durch Abnehmen des Aufsatzes besonders einfach und schnell der Entleerung zugeführt werden. Des Weiteren lässt sich der Aufsatz so problemlos auch auf andere Behälter aufsetzen und/oder nach Erfassung von Gegenständen einer Sterilisation zuführen, was die Einsatzbereitschaft der Erfindung erhöht.

Eine weitere besonders vorteilhafte Ausführung der erfindungsgemäßen Vorrichtung betrifft eine Kamera, deren Blickfeld den Bereich der Öffnung des Behälters erfasst und die in der Lage ist, Gegenstände, die dem Öffnungsbereich des Behälters und/oder Kompartiment zugeführt werden, z.B. anhand ihrer Formeigenschaft eindeutig zu identifizieren. So können vorteilhaft auch Gegenstände, die beispielsweise nicht mit einem Datenträger versehen sind, erfasst und dokumentiert werden. Darüber hinaus besteht die Möglichkeit, dass Gegenstände, deren Datenträger beispielsweise beschädigt sind und somit für einen gegebenen Lesekopf nicht mehr ausgelesen werden können, trotzdem identifiziert werden können. Die Wahrscheinlichkeit, daß ein mit einem Datenträger versehenen Gegenstand nicht gelesen werden kann, wird durch diese Weiterbildung abermals verringert, was die Qualität der Dokumentation weiter verbessert.

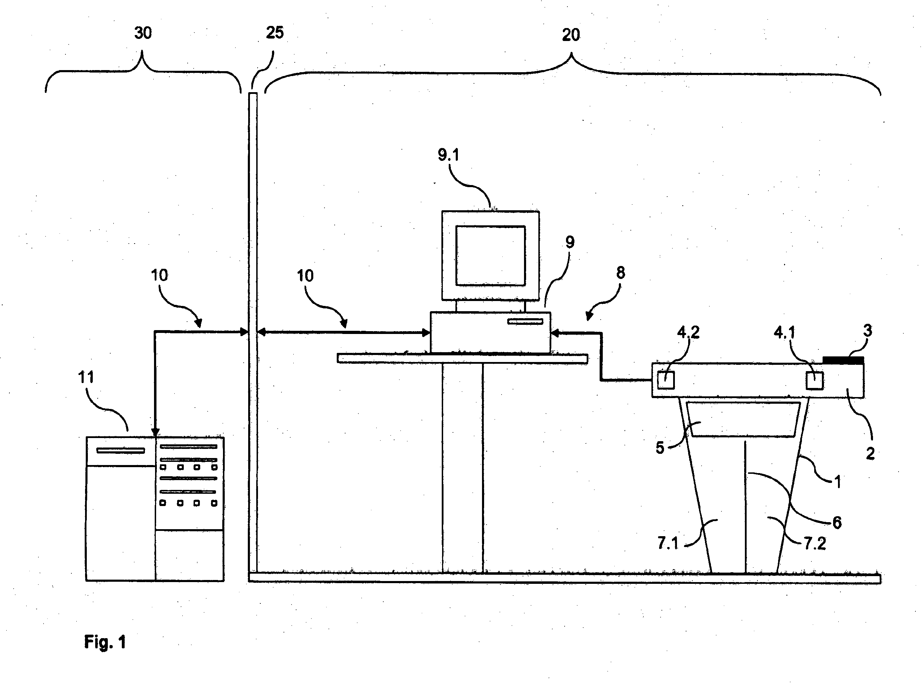

Eine beispielhafte Ausführungsform der erfindungsgemäßen Vorrichtung sowie das technische Umfeld der Erfindung werden nun anhand der Figur 1 näher erläutert. Die Erfindung ist nicht auf diese dargestellte Ausführung beschränkt.

Hierin zeigt Fig. 1 einen Operationssaal 20, dessen steriler Bereich durch eine Wand 25 vom nicht sterilen Bereich 30 getrennt ist. Weiterhin zeigt Fig. 1 eine auf dem Boden des Operationssaales 20 stehende Vorrichtung zur Aufnahme von zu entsorgenden Gegenständen für einen Operationssaal 20. Die Vorrichtung zeigt/weist einen Zweikammerbehälter 1 mit einer im Bereich der Öffnung des Behälters 1 angeordneten Identifizierungseinheit 2 auf sowie eine Sortiervorrichtung 5. Deren Positionierung ist unterhalb der Identifizierungseinheit 2 gewählt.

Die Identifizierungseinheit 2 ist auf dem oberen Rand des Behälters 1 in einen abnehmbaren ringförmigen Aufsatz integriert angeordnet, so dass dessen Öffnung in die Öffnung des Behälters 1 übergeht. Sie weist einen ersten Lesekopf 3, einen zweiten 4.1 und einen dritten Lesekopf 4.2 auf. Sie sind zum Detektieren von zu entsorgenden Gegenständen und zum Auslesen von Informationen aus Datenträgern, die mit den Gegenständen verbunden sind, ausgelegt, sobald diese in ihre Detektionsbereiche eingebracht werden. Die Datenträger enthalten Informationen, die zur individuellen Identifikation dienen, und sind an dem jeweiligen Gegenstand angebracht.

Die Leseköpfe 4.1 und 4.2 sind einander gegenüber so angeordnet, dass sich ihre Detektionsbereiche in der Fig. 1 in nicht näher dargestellten Art und Weise überlappen und den gesamten Öffnungsbereich des Behälters 1 abdecken.

Aufgrund dieser Anordnung von zwei Leseköpfen in einer gegenüberliegenden Position ist sicher gestellt, dass wenn ein Lesekopf den Datenträger nicht erfassen und auslesen kann, beispielsweise bei einer Abdeckung des Datenträgers, mindestens der andere Lesekopf dazu in der Lage ist.

Die dargestellten Leseköpfe 3, 4.1 und 4.2 lesen in der Regel die Barcode- und/oder die RFID-Identifizierungsdaten von Datenträgern aus, die in die Reichweite ihrer Detektionsbereiche eingebracht werden und sind entsprechend als Barcode- bzw. RFID-Lesegerät ausgelegt.

Weiter zeigt die Fig. 1 einen Zweikammerbehälter, der einen im Wesentlichen trapezförmigen Längsschnitt aufweist. Das Innere des Behälters 1 ist in seinem unteren Bereich in zwei Kompartimente 7.1 und 7.2 aufgeteilt, die von einer sich vom Boden des Behälters nach oben hin erstreckenden Trennwand 6 von einander getrennt sind. Mit Hilfe einer Sortiereinheit 5 erfolgt eine Sortierung der zugeführten Gegenstände. Das erste 7.1 und zweite Kompartiment 7.2 dient der Aufnahme selektiv sortierter Gegenstände, die sich in ihren Produkteigenschaften beispielsweise ihrem Werkstoff und/oder Funktion unterscheiden. Die Trennwand 6 sorgt dafür, dass sich die nach unterschiedlichen Produkteigenschaften im Behälter sortierten Gegenstände nicht mehr vermengen können.

Der Identifizierungseinheit 2 ist die Sortiereinheit 5 nachgeschaltet, die im Öffnungsbereich angeordnet, jedoch von ihrer Abmessungen her kleiner ist. Durch die Stellung eines Leitelements wird bestimmt, in welches Kompartiment die sortierten Gegenstände gelangen. Das Sortieren der Gegenstände kann dabei beispielsweise mittels einer winkelprofilartigen Weiche als Leitelement bewirkt werden, die um eine Achse schwenkbar gelagert ist. Die Sortiervorrichtung 5 erhält dabei Informationen, die von der Identifizierungseinheit 2 aus den Datenträgern der Gegenstände ausgelesen wurden und die Angaben über die Sortenzugehörigkeit eines Gegenstandes enthalten oder zulassen. Entsprechend der Sortieraufgabe wird der Gegenstand dann abgelenkt und ausgetragen.

Der Behälter 1 weist zwei Kompartimente 7.1 und 7.2 auf, die nebeneinander angeordnet sind. Die abgelenkten Gegenstände werden entsprechend ihrer Sortenzugehörigkeit, weswegen sie aussortiert wurden, nach links in das erste Kompartiment 7.1 geschleust, oder nach rechts in das zweite Kompartiment 7.2.

Die Identifizierungseinheit 2 und die Auswerteeinheit 9 sind für den Austausch von Daten über eine Kommunikationsverbindung 8 miteinander verbunden. Der Datentransfer kann dabei durch ein Kabel zwischen der Identifizierungseinheit 2 und der Auswerteeinheit 9 oder durch eine IR-Verbindung oder Bluetooth Verbindung, erfolgen.

In der Auswerteeinheit 9 laufen alle von der Identifizierungseinheit 2 ermittelten und ausgelesenen Daten zusammen. Die übertragenen Daten werden zunächst gespeichert, um anschließend wieder ausgelesen und weiter ausgewertet bzw. verarbeiten werden zu können.

Die Auswertung der eingelesenen Daten kann beispielsweise auf Grundlage von Auswertungsprozeduren abgewickelt werden, die gegebenenfalls keine zusätzlichen besonderen Eingabeoperationen des Anwenders erfordern. So können nach Eintreffen der Daten beispielsweise Gesamtinformationen der zugeführten Gegenstände bestimmt werden oder Gegenstände gemäß ihrer Sortenzugehörigkeit tabellarisch gruppiert und/oder angezeigt werden. Es ist möglich, die Anzeige kontinuierlich aufgrund eines laufenden Eingangs von Daten zu verändern und zu dokumentieren.

Auf dem Monitor 9.1 der Auswerteeinheit 9, können die eingelesenen Daten als Rohdatensatz und/oder in ihrer ausgewerteten Form angezeigt werden.

Es wird also letztlich angegeben, um welche Art von chirurgischem Instrument bzw. um welches medizinische Verbrauchsmaterial es sich vorliegend handelt. Weitere erfasste Daten wie beispielsweise Datum und Uhrzeit der Datenerfassung können bedarfsweise natürlich auch angezeigt werden.

An der Auswerteeinheit 9 können diese Daten durch manuelle und/oder sprachgesteuerte Eingabe von weiteren Daten wie beispielsweise Patientenidentifizierungsdaten oder Angaben zum Operationssaal 20 ergänzt werden. So können die im Bereich der Identifizierungseinheit 2 erfassten und in der Auswerteeinheit 9 gespeicherten Daten eindeutig einem Patienten und/oder einem Operationssaal 20 zugeordnet werden, aus dem sie stammen. Dies ist insbesondere dann wichtig, wenn eine Auswerteeinheit 9 verschiedenen Operationssälen 20 zugeordnet ist und/oder bei verschiedenen Eingriffen genutzt wird.

Die durch die Auswerteeinheit 9 verarbeiteten Daten und Auswerteergebnisse werden dann an einen zentralen Rechner 11 des Krankenhauses, der in einem nicht sterilen Bereich 30 des Krankenhauses aufgestellt ist, zur Verfügung gestellt. Die Kommunikationsleitung 10 zwischen der Auswerteeinheit 9 und dem zentralen Rechner 11 des Krankenhauses ist dabei als Glasfaserkabel ausgebildet. Die Datenübertragung kann dabei auch unter Einbindung des Internets als Datentransfersystem abgewickelt werden. In diesem Fall kann zusätzlich ein Update der Firmen/Software der Auswerteeinheit und/oder der Identifizierungseinheit über die Auswerteeinheit erfolgen, so dass der Benutzer die jeweils aktuellen Softwarestände nutzen kann. Die Pflege und Erweiterung der Datenerfassungs- und/oder Datenausauswertemöglichkeit im Rahmen der bestehenden Hardware ist somit einfach, kostengünstig und schnell möglich.

Auf dem zentralen Rechner 11 des Krankenhauses ist eine Datenbank angeordnet, in der neben der erfassten bzw. ergänzten Daten auch weitere Daten abgespeichert und enthalten sind, mittels derer sich insbesondere der Materialfluss in einem Operationssaal 20 darstellen lässt.

Mit dem Eintritt eines Gegenstandes in das Sterilgutlager des Krankenhauses werden dabei verschiedene kennzeichnende Daten eines jeden Gegenstandes registriert und in die Datenbank abgelegt. Dies erfolgt entweder manuell oder halb- bzw. vollautomatisch. Aus diesen Daten der registrierten Gegenstände kann dann ein Zuordnungsdatensatz erzeugt werden. Dieser Zuordnungsdatensatz ermöglicht unter anderem eine spätere Zuordnung von Rohdaten und Auswertungsergebnissen zu einem jeweiligen Operationssaal 20 bzw. die Identifikation von durch die erfindungsgemäße Vorrichtung erfassten und zu entsorgenden Gegenständen anhand der ausgelesenen Daten aus den Datenträgern. Auf Basis dieser Dokumentationen ist es möglich bei laufendem Verbrauch von Gegenständen den Materialfluss innerhalb des Krankenhauses darzustellen, und die Entsorgungslogistik, die Verwertung oder die Beseitigung des Gegenstandes zu vereinfachen.

### Bezugszeichen

- 1: Behälter
- 2: Identifizierungseinheit
- 3: Lesekopf, Barcodelesegerät
- 4.1: Lesekopf
- 4.2: Lesekopf
- 5: Sortieranlage
- 6: Trennwand
- 7.1: Kompartiment
- 7.2: Kompartiment
- 8: Kommunikationsverbindung
- 9: PC Arbeitsplatz
- 9.1: Bildschirm
- 10: Kommunikationsleitung
- 11: Zentraler Rechner des Krankenhauses
- 20: Operationssaal
- 25: Wand
- 30: Nicht sterile Bereich

## Patentansprüche

1. Vorrichtung zur Aufnahme von zu entsorgenden Gegenständen für einen Operationssaal (20), wobei sie einen mit einer Öffnung versehenen Behälter (1) aufweist, wobei im Bereich der Öffnung eine Identifizierungseinheit (2), die mit wenigstens einem Lesekopf (3, 4.1, 4.2) versehen ist, der geeignet ist, die Datenträger zu erfassen und die Daten des Datenträgers auszulesen, angeordnet ist, mittels der mit einem oder mehreren Datenträgern versehene Gegenstände erfasst werden können, wobei durch die Identifizierungseinheit die Erfassung ausschließlich der tatsächlich zu entsorgenden Gegenstände gewährleistet wird, und dass der wenigstens eine Lesekopf (3, 4.1, 4.2) mit seinem Detektionsbereich die gesamte Öffnung des Behälters (1) so abdeckt, das mit Datenträgern versehene Gegenstände vollständig erfasst werden, **dadurch gekennzeichnet, dass** die Identifizierungseinheit (2) mehrere, vorzugsweise zwei Leseköpfe (4.1, 4.2) aufweist und diese so von einander beabstandet und angeordnet sind, dass sich die Detektionsbereiche der Leseköpfe (4.1, 4.2) überlappen.

2. Vorrichtung nach einem der voranstehenden Ansprüche, **dadurch gekennzeichnet, dass** wenigstens ein Lesekopf (3, 4.1 und 4.2) ein Barcodelesegerät und/oder RFID-Lesegerät ist und Datenträger auslesen kann, die als Barcodes und/oder RFID-Tags ausgebildet sind.

3. Vorrichtung nach einem der voranstehenden Ansprüche, **dadurch gekennzeichnet, dass** die Identifizierungseinheit (2) über eine Kommunikationsverbindung (8) mit einer abgesetzten Auswerteeinheit (9) insbesondere außerhalb des Operationssaales (20) verbindbar ist.

4. Vorrichtung nach Anspruch 4, **dadurch gekennzeichnet, dass** die abgesetzte Auswerteeinheit (9) mit einer zentralen Datenbank (11) zur Darstellung des Materialflusses verbunden ist.

5. Vorrichtung nach einem der voranstehenden Ansprüche, **dadurch gekennzeichnet, dass** die Vorrichtung eine Verbindungseinrichtung aufweist, mit mindestens einem Dateneingang
für den Anschluss eines Handgerätes zum Eingeben von gegenstandsbezogenen Daten und/oder zum Auslesen der durch die Identifizierungseinheit erfassten, ausgewerteten oder gespeicherten Daten
und/oder für den Anschluss eines Handlesegerätes zum Erfassen und Auslesen von Datenträgern, die mit zu entsorgenden Gegenstände verbunden sind.

6. Vorrichtung nach einem der voranstehenden Ansprüche, **dadurch gekennzeichnet, dass** sie mit einer Füllzustandsdetektionseinrichtung versehen ist, die das Volumen und/oder das Gewicht eingebrachter Gegenstände erfasst und insbesondere daraus das Gesamtgewicht und/oder Gesamtvolumen aller eingebrachten Gegenstände berechnet, ausgibt oder in einer Speichervorrichtung abspeichert.

7. Vorrichtung nach einem der voranstehenden Ansprüche, **dadurch gekennzeichnet, dass** der Innenraum des Behälters (1) eine oder mehrere Trennwände (6) so aufweist, das dieser in einzelne Kompartimente (7.1 und 7.2), zur Aufnahme von verschiedenen Gegenständen, unterteilt ist.

8. Vorrichtung nach einem der voranstehenden Ansprüche, **dadurch gekennzeichnet, dass** sie einen oder mehrere Deckel aufweist, um die Öffnung des mindestens einen Behälters (1) und/oder die Öffnung eines Kompartiments (7.1 und 7.2) abzudecken, wobei ein automatisches Öffnen und Schließen wenigstens eines Deckels des Behälters (1) und/oder Kompartiments (7.1 und 7.2) durch die Identifizierungseinheit gesteuert erfolgt.

9. Vorrichtung nach einem der voranstehenden Ansprüche, **dadurch gekennzeichnet, dass** im Öffnungsbereich eine bedarfsgesteuerte Sortiervorrichtung angeordnet ist, die, von der Identifizierungseinheit (2) gesteuert, einen zu entsorgenden Gegenstand anhand der erfassten Daten einem seiner Sortenzugehörigkeit entsprechenden Kompartiment zuführt.

10. Vorrichtung nach einem der voranstehenden Ansprüche, **dadurch gekennzeichnet, dass** ein ringförmiger Aufsatz auf den Behälter (1) vorgesehen ist, der die Identifizierungseinheit (2) beinhaltet und im Bereich der Öffnung des Behältnisses (1) abnehmbar und aufsetzbar angeordnet werden kann.

11. Vorrichtung nach einem der voranstehenden Ansprüche, **dadurch gekennzeichnet, dass** zusätzlich eine Kamera vorgesehen ist, die den Bereich der Öffnung erfasst und die geeignet ist, zu entsorgende Gegenstände zu identifizieren.

## Claims

1. Device for receiving objects to be disposed of, for an operating theatre (20), said device comprising a container (1) on which an opening is provided, wherein an identification unit (2) is arranged in the region of the opening and provided with at least one read head (3, 4.1, 4.2) suitable for registering the data media and reading the data from the data medium, wherein said identification unit can be used to register objects provided with one or more data media, with the identification unit ensuring the registration only of objects which are in fact to be disposed of, and the at least one read head (3, 4.1, 4.2) covers with the detection region thereof the whole opening of the container (1) such that objects provided with data media are completely registered, **characterized in that** the identification unit (2) has a plurality of, preferably two, read heads (4.1, 4.2) and these are spaced apart and arranged in such a way that the detection regions of the read heads (4.1, 4.2) overlap.

2. Device according to one of the preceding claims, **characterized in that** at least one read head (3, 4.1 and 4.2) is a barcode reading instrument and/or an RFID reading instrument and can read data media which are embodied as barcodes and/or RFID tags.

3. Device according to one of the preceding claims, **characterized in that** the identification unit (2) is connectable to a remote evaluation unit (9), situated outside of the operating theatre (20) in particular, by means of a communication link (8).

4. Device according to Claim 4, **characterized in that** the remote evaluation unit (9) is connected to a central database (11) for illustrating the material flow.

5. Device according to one of the preceding claims, **characterized in that** the device comprises a connection apparatus, having at least one data input for connecting a hand-held instrument for entering object-related data and/or for reading the data registered, evaluated or stored by the identification unit
and/or for connecting a hand-held reading instrument for registering and reading data media which are connected to objects to be disposed of.

6. Device according to one of the preceding claims, **characterized in that** it is provided with a filling state detection apparatus, which registers the volume and/or the weight of introduced objects and, in particular, calculates, outputs or stores in a storage device the overall weight and/or the overall volume of all introduced objects therefrom.

7. Device according to one of the preceding claims, **characterized in that** the interior of the container (1) has one or more separating walls (6) such that said interior is subdivided into individual compartments (7.1 and 7.2) for holding different objects.

8. Device according to one of the preceding claims, **characterized in that** it has one or more covers in order to cover the opening of the at least one container (1) and/or the opening of a compartment (7.1 and 7.2), with automated opening and closing of at least one of the covers of the container (1) and/or compartments (7.1 and 7.2) is carried out, controlled by the identification unit.

9. Device according to one of the preceding claims, **characterized in that** a demand-driven sorting device is arranged in the opening region, which sorting device, controlled by the identification unit (2), feeds an object to be disposed of to a compartment corresponding to its type-membership on the basis of the registered data.

10. Device according to one of the preceding claims, **characterized in that** a ring-shaped attachment is provided on the container (1), which attachment contains the identification unit (2) and can be arranged in a removable and replaceable manner in the region of the opening of the container (1).

11. Device according to one of the preceding claims, **characterized in that** provision is additionally made for a camera, which registers the region of the opening and is suitable to identify objects to be disposed of.

## Revendications

1. Dispositif de réception d'objets à éliminer dans une salle d'opération (20), le dispositif présentant un récipient (1) doté d'une ouverture,
une unité d'identification (2) dotée d'au moins une tête de lecture (3, 4.1, 4.2) qui permet de saisir des supports de données et de lire les données sur le support de données étant disposée au niveau de l'ouverture et permettant de saisir des objets dotés d'un ou de plusieurs supports de données,
l'unité d'identification assurant exclusivement la saisie des objets qui doivent effectivement être éliminés, et
la ou les têtes de lecture (3, 4.1, 4.2) recouvrant par leur zone de détection la totalité de l'ouverture du récipient (1) de telle sorte que les objets dotés de supports de données soient saisis complètement,
**caractérisé en ce que**
l'unité d'identification (2) présente plusieurs et de préférence deux têtes de lecture (4.1, 4.2) et
**en ce que** ces dernières sont disposées à une distance mutuelle telle que les zones de détection des têtes de lecture (4.1, 4.2) se superposent.

2. Dispositif selon l'une des revendications précédentes, **caractérisé en ce qu'**au moins une tête de lecture (3, 4.1 et 4.2) est un appareil de lecture de codes à barres et/ou un appareil de lecture RFID et est en mesure de lire des supports de données configurés comme codes à barres et/ou étiquettes RFID.

3. Dispositif selon l'une des revendications précédentes, **caractérisé en ce que** l'unité d'identification (2) peut être reliée par une liaison de communication (8) à une unité d'évaluation (9) située à distance et en particulier à l'extérieur de la salle d'opération (20).

4. Dispositif selon la revendication 4, **caractérisé en ce que** l'unité d'évaluation (9) à distance est raccordée à une base de données centrale (11) permettant d'établir le flux de matière.

5. Dispositif selon l'une des revendications précédentes, **caractérisé en ce que** le dispositif est doté d'un dispositif de liaison présentant au moins une entrée de données pouvant être raccordée à un appareil manuel d'introduction de données concernant les objets et/ou de lecture des données saisies par l'unité d'identification, évaluées ou conservées en mémoire et/ou être raccordée à un appareil manuel de lecture permettant de saisir et de lire des supports de données raccordés aux objets à éliminer.

6. Dispositif selon l'une des revendications précédentes, **caractérisé en ce qu'**il est doté d'un dispositif de détection du niveau de remplissage qui saisit le volume et/ou le poids des objets introduits et en particulier calcule à partir de cela le poids total et/ou le volume total de tous les objets introduits, les délivre ou les conserve dans un dispositif de mémoire.

7. Dispositif selon l'une des revendications précédentes, **caractérisé en ce que** l'espace intérieur du récipient (1) présente une ou plusieurs cloisons de séparation (6), de telle sorte que cet espace intérieur est divisé en différents compartiments (7.1 et 7.2) permettant de reprendre différents objets.

8. Dispositif selon l'une des revendications précédentes, **caractérisé en ce qu'**il présente un ou plusieurs couvercles qui recouvrent l'ouverture du ou des récipients (1) et/ou l'ouverture d'un compartiment (7.1 et 7.2), une ouverture et une fermeture automatique d'au moins un couvercle du récipient (1) et/ou du compartiment (7.1 et 7.2) s'effectuant sous la commande de l'unité d'identification.

9. Dispositif selon l'une des revendications précédentes, **caractérisé en ce qu'**un dispositif de tri en fonction des besoins est disposé au niveau de l'ouverture et apporte un objet à éliminer à un compartiment qui correspond à sa classe de tri à l'aide des données saisies, sous la commande de l'unité d'identification (2).

10. Dispositif selon l'une des revendications précédentes, **caractérisé en ce qu'**un appendice annulaire qui contient l'unité d'identification (2) est prévu sur le récipient (1) et peut être disposé de manière à pouvoir être enlevé ou placé au niveau de l'ouverture du récipient (1).

11. Dispositif selon l'une des revendications précédentes, **caractérisé en ce qu'**il présente en outre une caméra qui saisit la zone de l'ouverture et qui permet d'identifier les objets à éliminer.
